# EUROPEAN PATENT APPLICATION

(11) **EP 2 711 062 A1**
(43) Date of publication of application: **26.03.2014**
(21) Application number: 13185108.1
(22) Date of filing: 19.09.2013
(51) Int. Cl.: B01D 1/28, B01D 3/00, B01D 3/42

(54) **Stripper for separating process in aromatic component processing apparatus and method for operating the same**

(30) Priority: 21.09.2012 JP 2012208129
(71) Applicant: Toyo Engineering Corporation, Chiyoda-ku, Tokyo 100-6511 (JP)
(72) Inventor: Wakabayashi, Toshihiro, CHIBA, Chiba 275-0024 (JP); Nakao, Takato, CHIBA, Chiba 275-0024 (JP)
(74) Representative: Minoja, Fabrizio

(57) **Abstract**

The present invention provides a method for operating a stripper that is provided for a separating process in an aromatic component processing apparatus in which the stripper separates a component that is lighter than an aromatic component from the aromatic component via a distillation operation. In the method, using a HIDiC as the stripper, the pressure of the column top of rectifying section (201) of the HIDiC is determined, and the pressure of the column top of stripping section (202) of the HIDiC is set to be lower than the pressure of the column top of rectifying section (201).

## Description

This application is based upon and claims the benefit of priority from Japanese patent application No. 2012-208129, filed on September 21, 2012, the disclosure of which is incorporated herein in its entirety by reference.

### BACKGROUND OF THE INVENTION

### 1. Field of the Invention

The present invention relates to a stripper for a separating process in an aromatic component manufacturing apparatus and a method for operating the same, in particular, an operation method when separating components that are lighter than aromatic components from the aromatic components by means of a distillation operation using a stripper.

### 2. Description of the Related Art

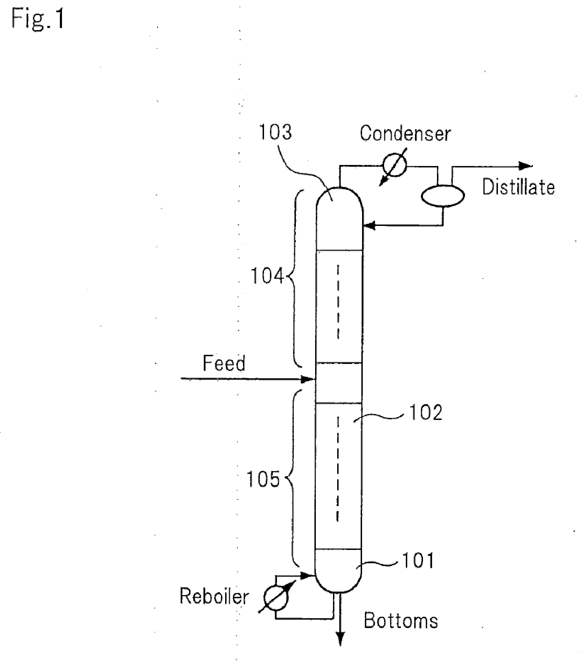

In a separating process in aromatic component processing apparatuses, an apparatus, called stripper, that separates components that are lighter than aromatic components from the aromatic components may be provided. A stripper separates light components from aromatic components by means of a distillation operation. For such stripper that performs a distillation operation, conventionally, the distillation apparatus shown in FIG. 1 is generally used. The distillation apparatus, which is a column built in a vertical direction, includes column bottom 101, trayed section (or packed bed section) 102 and column top 103, and trayed section (or packed bed section) 102 consists of rectifying section 104 on the upper side and stripping section 105 on the lower side with a raw material supply position as the boundary therebetween.

A stripper that includes such distillation apparatus is operated so that the operating pressure is around 600 kPa (absolute pressure) (hereinafter referred to as "A") or more at the column top in order to provide a temperature that allows vapor in the column top that contains components lighter than aromatic components to be condensed by cooling water or the atmosphere.

Distillation separation is a unit operation widely applied to industrial processes in general, but consumes a large amount of energy. In the industrial field, therefore, studies have been conducted on a distillation system that can save energy. Such studies have brought about development of a heat integrated distillation column (hereinafter, HIDiC) as a distillation apparatus that saves much energy.

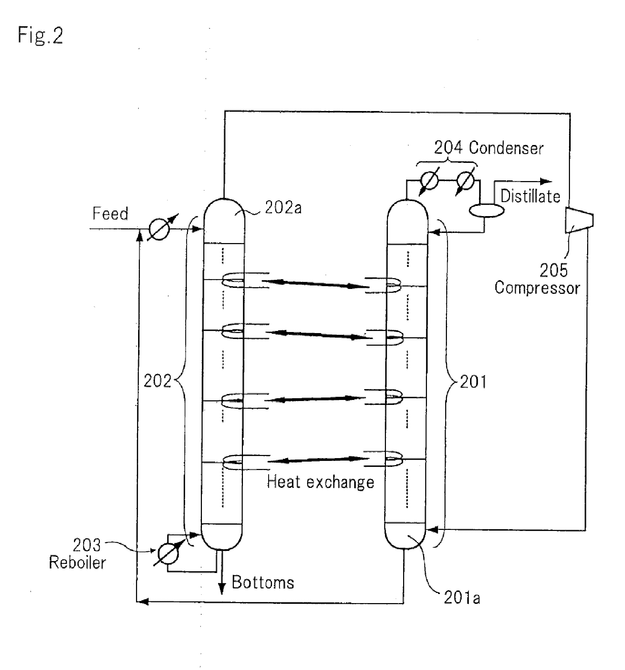

As shown in FIG. 2, a basic system of a HIDiC has a structure where rectifying section 201 and stripping section 202 are provided so that they are separate from each other. The operating pressure of rectifying section 201 is set to be higher than that of stripping section 202 so that the operating temperature of rectifying section 201 is higher than that of stripping section 202. This enables reduction in the amount of heat supplied to reboiler 203 because heat transfer occurs from rectifying section 201 to stripping section 202 if there is a heat-exchange surface therebetween. Heat of the rectifying section moves to the stripping section, and hence the amount of heat removed by condenser 204 can also be reduced. As a result, a high energy saving distillation apparatus can be provided.

In order to put such HIDiC into practical use, apparatus configurations such as indicated in, e.g., JP2004-16928A and JP Patent No. 4803470 have been proposed.

As shown in FIG. 2, a HIDiC is configured in such a manner that rectifying section 201 and stripping section 202 are provided in separate sections (columns), vapor is removed from column top 202a of stripping section 202 and compressed by compressor 205, and the vapor having a high temperature and a high pressure as a result of the compression is introduced into column bottom 201a of rectifying section 201. Accordingly, the operating pressure and operating temperature of rectifying section 201 are higher than those of stripping section 202. Also, as stated above, the HIDiC is an apparatus that can be expected to achieve enhanced energy saving compared with the conventional distillation apparatus shown in FIG. 1.

One may conceive of using a HIDiC for a stripper for a separating process in an aromatic component processing apparatus; however, in this case, there are the following problems to be solved.

In general, when separation is performed by means of distillation, the conventional distillation apparatus shown in FIG. 1 is operated at an operating pressure (101 kPa(A) to 150 kPa(A)) that is atmospheric pressure or slightly larger than atmospheric pressure. If the conventional distillation apparatus is replaced with a HIDiC, the operating pressure that is equal to or is slightly higher than atmospheric pressure in the conventional distillation apparatus is ordinarily set as the operating pressure on the stripping section side of the HIDiC. This is because, if the operating pressure on the rectifying section side of the HIDiC is made to be the pressure that is close to atmospheric pressure, it is necessary to provide a decompression apparatus that makes the inside of the stripping section have a pressure equal to or smaller than atmospheric pressure for the stripping section, in order to make the operating pressure of the stripping section of the HIDiC be lower than that of the rectifying section side of the HIDiC.

However, as stated above, if the conventional distillation apparatus is provided as a stripper for the separating process in an aromatic component processing apparatus, the operating pressure at the column top of the stripper is around 600 kPa(A) or higher. If such pressure is the operating pressure on the stripping section side of a HIDiC, the operating pressure on the rectifying section side of the HIDiC becomes very large relative to the operating pressure on the stripping section side. In a HIDiC, vapor removed from the column top of a stripping section thereof is compressed to supply high-temperature, high-pressure vapor to the column bottom of a rectifying section thereof; however, there is a limit on the outlet temperature that can be provided by current fluid compressors, and thus, a HIDiC cannot be employed if pressure of around 600 kPa(A) or more is set as the operating pressure on the stripping section side of the HIDiC.

Furthermore, in the first place, high pressure in a distillation operation directly leads to a decrease in relative volatility, which provides a driving force for distillation separation, and consequently, the heat duty on a reboiler at the column bottom increases on the stripping section side of the HIDiC, resulting in a large operation cost. Meanwhile, decreasing the operating pressure on the stripping section side decreases the operating temperature so that it is lower than that of the stripping section of the conventional distillation apparatus, enabling a heat source of the reboiler at the column bottom to be changed from conventionally-used medium-pressure steam to low-pressure steam. Such change brings a cost advantage.

### SUMMARY OF THE INVENTION

An object of the present invention to solve the aforementioned problems that occur when the conventional distillation apparatus that is provided as a stripper for a separating process in an aromatic component processing apparatus is replaced with a HIDiC.

An aspect of the present invention relates to a method for operating a stripper that is provided for a separating process in an aromatic component processing apparatus in which the stripper separates a component that is lighter than an aromatic component from the aromatic component via a distillation operation. In the operation method, a HIDiC is used as the stripper, first, the pressure of the column top of a rectifying section of the HIDiC is determined, and then the pressure of the column top of the stripping section of the HIDiC is set to be lower than the pressure of the column top of the rectifying section.

Also, another aspect of the present invention relates to a stripper that is provided for a separating process in an aromatic component processing apparatus in which the stripper separates a component that is lighter than the aromatic component from the aromatic component via a distillation operation. The stripper includes a distillation apparatus that includes a HIDiC, and the operating pressure range can be controlled to be 600 to 1000 kP(A) at the column top of the rectifying section of the HIDiC and to be 150 to 300 kPa(A) at the column top of the stripping section of the HIDiC.

The present invention enables employment of the HIDiC as a stripper for the separating process in an aromatic component processing apparatus. Also, the stripper includes a HIDiC, enabling enhanced energy saving and reduction in operating cost.

The above and other objects, features and advantages of the present invention will become apparent from the following description with reference to the accompanying drawings which illustrate examples of the present invention.

### BRIEF DESCRIPTION OF THE DRAWINGS

FIG. 1 is a diagram showing a configuration of a distillation apparatus;
FIG. 2 is a diagram showing a conceptual configuration of a HIDiC;
FIG. 3A is a diagram showing a conceptual configuration of a stripper (HIDiC type) for a separating process in an aromatic component processing apparatus, according to the present invention; and
FIG. 3B is a diagram showing a stripper using a conventional distillation apparatus, which is compared with that of the HIDiC type.

### DETAILED DESCRIPTION OF THE PREFERRED EMBODIMENTS

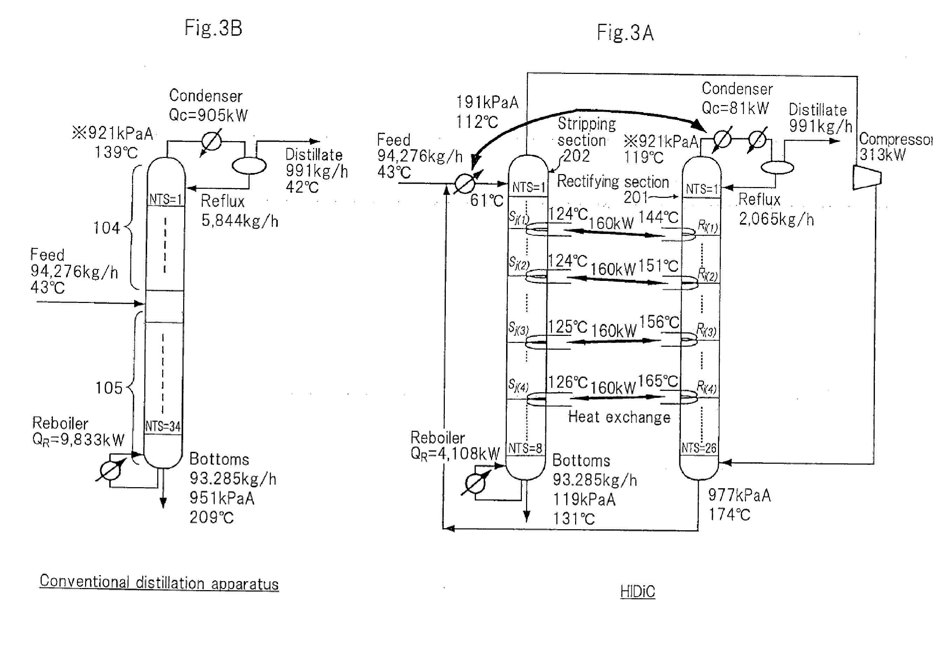

An embodiment of the present invention will be described with reference to the drawings. FIG. 3A is a diagram showing a conceptual configuration of a stripper (HIDiC-employed type) for a separating process in an aromatic component processing apparatus, according to the present invention, and FIG. 3B is a diagram showing a stripper using a conventional distillation apparatus in comparison with the HIDiC-type apparatus.

First, a stripper that is provided for a separating process in an aromatic component processing apparatus will be described in detail. For example, the stream from an outlet of a reactor for an aromatic component disproportionation process contains traces of hydrogen and light hydrocarbons (e.g., ethane, propane and butane) in addition to benzene, toluene and C8 aromatic components (ethylbenzene, styrene, paraxylene, meta-xylene and ortho-xylene), which are main components. It is necessary to first separate the hydrogen and the light hydrocarbon from the stream and then separate the stream into benzene, toluene and xylenes in the following distillation operation. The stripper plays a role in the light component separation.

If the conventional distillation apparatus shown in FIG. 3B is used as a stripper, a part of a gas in the column top is condensed in a condenser at the column top, and the condensate is recycled into the column body as reflux, and the remaining gas-phase light mixture is removed to the outside of the column. At this time, a part of benzene cannot completely be separated from the light mixture and is removed together with the light mixture. In order to achieve a benzene collection rate of around 99% or more at the column bottom in such partial distillate condensation, if it is assumed that air or cooling water is used for condensation, an operating pressure of around 600 to 1000 kPa(A) is required. Along with that, the temperature of the column bottom is determined by a calculation that is typically around 200 to 220°C. In the present embodiment, as shown in FIG. 3B, the pressure of the column top is 921 kPa(A), the temperature of the column top is 139°C, the pressure of the column bottom is 951 kPa(A) and the temperature of the column bottom is 209°C.

When a HIDiC is employed for such stripper, if the operating pressure on the stripping section side of the HIDiC is around 600 to 1000 kPa(A), it is necessary to set the operating pressure on the rectifying section side of the HIDiC to be higher than 600 to 1000 kPa(A). Along with the operating pressure, the temperature at the outlet of a compressor, which corresponds to the temperature of the column bottom of the rectifying section, is around 220 to 240°C when taking into consideration heating in a superheated state. Currently-developed compressors that accept hydrocarbons do not cover such a high temperature range, and thus, a HIDiC cannot actually be employed.

Therefore, as shown in FIG. 3A, the operating pressure on the rectifying section 201 side of the HIDiC is set so that the pressure at the column top of the rectifying section of the HIDiC becomes the pressure at the column top of the conventional distillation apparatus (921 kPa(A)). With reference to the pressure at the rectifying section, the operating pressure on the stripping section 202 side is set to be lower than 921 kPa(A).

As described above, as a result of the operating pressure of stripping section 202 being set to be low in comparison with the conventional distillation apparatus, the operating temperature of stripping section 202 can be reduced so that it is low. Accordingly, the temperature at the column bottom of stripping section 202 is also decreased so that it is lower than that of the conventional distillation apparatus, and reaches a temperature of around 130°C, which enables use of low-pressure steam.

Furthermore, since the pressure at the column top of the rectifying section of the HIDiC is made to be the pressure at the column top of the conventional distillation apparatus (921 kPa(A)), the above-mentioned temperature problem at the outlet of the compressor is eliminated.

In the present embodiment, as shown in FIG. 3A, the pressure of the column top of the rectifying section is 921 kPa(A), the temperature of the column top of the rectifying section is 119°C, the pressure of the column bottom of the rectifying section is 977 kPa(A), and the temperature of the column bottom of the rectifying section is 174°C. Along with these, the pressure of the column top of the stripping section is 191 kPa(A), the temperature of the column top of the stripping section is 112°C, the pressure of the column bottom of the stripping section is 199 kPa(A), and the temperature of the column bottom of the stripping section is 131°C.

Here, the present inventors have concluded based on simulations that when a HIDiC is used as a stripper for a separating process in an aromatic component processing apparatus, if the operating pressure range of the HIDiC is 600 to 1000 kPa(A) at the column top of the rectifying section thereof, the operating pressure range is preferably 150 to 300 kPa(A) at the column top of the stripping section thereof.

While preferred embodiments of the present invention have been described using specific terms, such description is for illustrative purposes only, and it is to be understood that changes and variations may be made without departing from the spirit or scope of the following claims.

## Claims

1. A method for operating a stripper provided for a separating process in an aromatic component processing apparatus in which said stripper separates a component that is lighter than an aromatic component from the aromatic component via a distillation operation, the method comprising,
using a heat integrated distillation column (HIDiC) as the stripper, determining a pressure of a column top of a rectifying section of the heat integrated distillation column and then setting a pressure of a column top of a stripping section of the heat integrated distillation column to be lower than the pressure of the column top of the rectifying section.

2. The method for operating a stripper according to claim 1, wherein the pressure of the column top of the rectifying section is set within a range of 600 to 1000 kPa(A) and the pressure of the column top of the stripping section is set within a range of 150 to 300 kPa(A).

3. A stripper provided for a separating process in an aromatic component processing apparatus in which said stripper separates a component that is lighter than an aromatic component from the aromatic component via a distillation operation, the stripper comprising a distillation apparatus that includes a heat integrated distillation column (HIDiC), wherein an operating pressure range can be controlled to be 600 to 1000 kPa(A) at a column top of a rectifying section of the heat integrated distillation column and to be 150 to 300 kPa(A) at a column top of a stripping section of the heat integrated distillation column.
